# EUROPÄISCHE PATENTSCHRIFT

(11) **EP 1 958 590 B1**
(45) Veröffentlichungstag und Bekanntmachung des Hinweises auf die Patenterteilung: **29.04.2015**
(21) Anmeldenummer: 08002588.5
(22) Anmeldetag: 13.02.2008
(51) Int. Cl.: A61F 2/06, B29C 41/08, B29C 41/38, B29C 33/48, B29C 41/42, B29C 53/30

(54) **Nicht gewebte Gefässprothese und Verfahren zu Ihrer Herstellung**
Non-woven graft and method for its manufacture
Prothèse vasculaire non tissée et son procédé de fabrication

(30) Priorität: 13.02.2007 DE 102007008185
(43) Veröffentlichungstag der Anmeldung: 20.08.2008
(73) Patentinhaber: Aesculap AG, 78532 Tuttlingen (DE)
(72) Erfinder: Goldman, Helmut, D-34119 Kassel (DE); Langanke, Dennis, D- 72074 Tübingen (DE); Probst, Dietmar, D-34212 Melsungen (DE)
(74) Vertreter: Patentanwälte Ruff, Wilhelm, Beier, Dauster & Partner

(56) Entgegenhaltungen:
- EP-A- 0 698 379
- WO-A-00/38591
- WO-A-2004/100827
- WO-A1-2006/054799
- WO-A2-2004/110304
- DE-A1- 10 162 821
- DE-B- 1 209 279
- DE-U1- 20 306 639
- FR-A- 1 538 078
- US-A- 2 446 281

## Beschreibung

Die Erfindung betrifft eine non woven Gefäßprothese.

Non woven Gefäßprothesen in Form eines porösen Schlauches sind bereits bekannt. Sie können aus expandiertem Polytetrafluorethylen (e-PTFE) hergestellt werden und besitzen je nach Wandstärke eine gute Durchmesserstabilität.

Eine non woven Gefäßprothese mit einer aus einem Vlies gebildeten Gefäßwandung ist aus der WO 2004/110304 A2 bekannt.

Non woven Gefäßprothesen können in geeigneter Weise auch durch Sprühvliesverfahren hergestellt werden, indem eine Lösung eines Polymers in einem leicht flüssigen Lösungsmittel durch Sprühtechnik auf einen Kern aufgesprüht wird. Das Lösungsmittel verflüchtigt sich beim Durchlaufen des Sprühweges, so dass sich auf dem Kern noch klebrige Polymerfasern abscheiden, die sich unter Bildung einer dreidimensionalen Faserstruktur miteinander verkleben. Der Vorteil einer solchen Sprühtechnik ist der, dass sich auch bereits gebogene Gefäßprothesen unter Verwendung eines entsprechend gebogenen Kerns herstellen lassen. Derartige bogenförmige non woven Gefäßprothesen sind beispielsweise in der DE-A-101 62 821.8 beschrieben.

Es kommt immer wieder vor, dass Gefäßprothesen benötigt werden, bei denen sich gebogene Abschnitte und gerade Abschnitte oder unterschiedlich gebogene Abschnitte abwechseln. Hier ist es schwierig, solche Gefäßprothesen aus vorgefertigten Einzelabschnitten zusammenzusetzen. Es ist deshalb wünschenswert, eine non woven Gefäßprothese herzustellen, die in der gewünschten Weise gebogen werden kann, ohne dass die Gefahr eines Kollabierens besteht.

Diese Aufgabe wird dadurch gelöst, dass eine non woven Gefäßprothese mit einer Plissierung in der Gefäßwandung bereitgestellt wird.

Plissierungen sind bei textilen Gefäßprothesen, insbesondere bei gewebten oder gewirkten Gefäßprothesen bekannt. Sie können durch Ausbildung von umlaufenden Querfalten, Kompaktieren der Falten in axialer Richtung und Fixieren der Querfalten geschaffen werden. Die Plissierung bei den textilen Gefäßprothesen besteht aus einer Vielzahl von eng aneinander liegenden ziehharmonikaartigen Falten, die relativ scharfe Kanten aufweisen. In der Regel liegt die Ganghöhe der wendelförmig verlaufenden Plissierung bei textilen Gefäßprothesen unter einem Millimeter.

Eine solche Ausbildung einer Plissierung ist bei non woven Gefäßprothesen nicht möglich, alleine schon wegen der dreidimensionalen Faserstruktur.

Gemäß der Erfindung ist die Plissierung vorzugsweise wellenförmig ausgebildet. Übergänge zwischen Wellenbergen und Wellentälern verlaufen zumindest an der äußeren Oberfläche weich. Im Gegensatz zu der Plissierung bei textilen Gefäßprothesen ist bei der Erfindung keine Kompaktierung in Längsrichtung vorgesehen. Dadurch ist die erfindungsgemäße Gefäßprothese in Längsrichtung auch nur geringfügig dehnbar und zwar nur im Rahmen der Elastizität des Wandungsmaterials. Die Dehnbarkeit liegt in der Regel bei den während der Implantation und im Körper auftretenden Längskräften bei maximal 10 %.

Die erfindungsgemäße Gefäßprothese hat eine sehr gute Durchmesserstabilität und lässt sich um enge Radien biegen, ohne dass die Gefahr eines Kollapses der Prothesenwand zu befürchten ist, im Gegensatz zu den entsprechenden non woven Gefäßprothesen ohne Plissierung.

Die erfindungsgemäße plissierte Gefäßprothese ist vorzugsweise porös ausgebildet, d.h. sie besitzt eine poröse Gefäßwandung. Diese kann, falls erwünscht, mit einer resorbierbaren Imprägnierung abgedichtet sein. Wie bei den bekannten non woven Gefäßprothesen besteht die erfindungsgemäße Prothese aus einem Vlies, insbesondere einem Sprühvlies. Erfindungsgemäß ist Polyurethan als Wandungsmaterial vorgesehen, wobei das Polyurethan ein thermoplastisches Polyurethan, d.h. lineares Polyurethan, ist, das in Lösungsmitteln löslich ist. Die als Luftdurchlässigkeit definierte Porosität liegt vorzugsweise bei 1 bis 150 ml Luft pro Quadratzentimeter pro Minute bei einer Druckdifferenz von 1,2 KPas.

Die Plissierung kann in Form von umlaufenden Rillen vorgesehen sein, eine wendelförmig entlang der Gefäßwandung verlaufende Plissierung ist jedoch bevorzugt. Vertiefungen in der Plissierung, insbesondere Wellentäler sind vorzugsweise als Rillen ausgebildet. Bei einer Ausführungsform der Erfindung werden die Vertiefungen von Einschnürungen gebildet. Im Bereich der Vertiefungen, insbesondere Einschnürungen, kann die Gefäßwandung eine dichtere Struktur aufweisen, insbesondere kompaktiert sein. Eine Verdichtung der Gefäßwandung im Bereich der Vertiefungen im Vergleich zur Wandungsstärke außerhalb der Vertiefungen um 10 bis 60 %, insbesondere 20 bis 50 %, ist bevorzugt. Somit ist das Wandungsmaterial bei einer Ausführungsform der Erfindung im Bereich der Vertiefungen in radialer Richtung kompaktiert und beträgt bevorzugt nur noch 40 bis 90 %, insbesondere 50 bis 80 %, der Wandstärke außerhalb der der Verdichtung.

Der Durchmesser der erfindungsgemäßen Gefäßprothese kann in üblichen Bereichen liegen. Der Innendurchmesser liegt vorzugsweise bei 2 bis 40, insbesondere 4 bis 12, mm. Gerade bei kleineren Innendurchmessern von weniger als 10 mm besitzt die erfindungsgemäße Gefäßprothese besonders günstige Eigenschaften.

Die Gefäßwandung kann eine Dicke von 0,2 bis 1 mm, insbesondere 0,4 bis 0,6 mm, besitzen. Die Differenz zwischen Wellenberg und Wellental der Plissierung, d.h. die Rillentiefe, liegt vorzugsweise bei 0,2 bis 1 mm, insbesondere 0,4 bis 0,6 mm. Der axiale Abstand von Erhöhung zu zu Erhöhung, insbesondere die Ganghöhe einer wendelförmigen Plissierung, liegt vorzugsweise im Bereich von 1 bis 5 mm, vorzugsweise 1,5 bis 3,5 mm, insbesondere 2 bis 3 mm. Bei Prothesen mit einem Innendurchmesser von kleiner 10 mm, liegt der axiale Abstand vorzugsweise im größeren Bereich, insbesondere über 2,5 mm, und bei Prothesen mit einem Innendurchmesser von 10 mm und mehr liegt der Abstand vorzugsweise im unteren Bereich, insbesondere unterhalb von 2,5 mm. Eine solche Ausgestaltung erlaubt hervorragende Durchmesserstabilitäten in gebogenem Zustand.

Die Erfindung betrifft auch ein Verfahren zur Herstellung der erfindungsgemäßen plissierten non woven Gefäßprothese. Zur Herstellung ist es vorgesehen, dass die Gefäßwandung an einem stabförmigen Kern mit einer der Plissierung entsprechenden gewellten Oberfläche abgeformt wird. Vorzugsweise wird ein stabförmiger Kern mit einer wendelförmig umlaufenden Wellung auf seiner Oberfläche eingesetzt, so dass die Gefäßprothese eine entsprechend wendelförmig verlaufende Plissierung aufweist.

Für die Abformung der Plissierung gibt es verschiedene Möglichkeiten. Bei einer Ausführungsform der Erfindung wird eine vorgefertigte, noch nicht plissierte non woven Gefäßprothese zur Ausbildung der Plissierung auf den stabförmigen Kern aufgeschoben. Die Plissierung wird dann durch querschnittsverjüngende, insbesondere thermische Fixierung ausgebildet. Die vorgefertigte Gefäßprothese kann einen Innendurchmesser aufweisen, der dem Außendurchmesser des stabförmigen Kerns entspricht oder geringfügig größer ist. Es ist auch möglich, eine vorgefertigte schlauchförmige Gefäßprothese unter Durchmesseraufweitung auf den Kern aufzuschieben. Besonders vorteilhaft ist eine vorgefertigte Gefäßprothese, die schrumpffähig ausgebildet ist und auf den gewellten Stab aufgeschrumpft werden kann.

Die Abformung der Plissierung kann durch bleibende Verjüngung der Gefäßprothese im Bereich der Wellentäler der Plissierung vorgenommen werden. Dies kann durch den zuvor erwähnten Schrumpfeffekt geschehen. Es ist auch möglich, eine Einschnürung der vorgefertigten Gefäßprothese im Bereich der Wellentäler vorzunehmen und diese durch geeignete Maßnahmen zu fixieren. Die Einschnürung kann dadurch vorgenommen werden, dass ein Faden entsprechend der Steigung der Wendel der Plissierung im Bereich der Vertiefungen um die schlauchförmige Gefäßprothese gewickelt und diese dabei in die Vertiefungen des stabförmigen Kerns eingedrückt wird, wonach die Fixierung erfolgt. Es kann auch eine Schrumpfung mit einer mechanischen Einschnürung kombiniert werden. Nach Fixierung der ausgebildeten Plissierung kann der Stab aus der plissierten Gefäßprothese herausgezogen werden.

Ein Trennen von Stab und plissierter Gefäßprothese kann dadurch erleichtert werden, dass die Oberfläche des Stabes mit einer Gleitschicht versehen wird. Eine solche Gleitschicht kann aus einer gleitenden plastischen Masse bestehen oder in Form einer folienartigen Zwischenschicht ausgebildet sein.

Bei einer Ausführungsform der Erfindung wird die non woven Gefäßprothese unmittelbar auf dem stabförmigen Kern hergestellt. Dies kann durch unmittelbare Herstellung der plissierten Gefäßwandung auf dem stabförmigen Kern erfolgen. Geeignet ist hierzu wiederum die Sprühtechnik, insbesondere die Sprühvliestechnik.

Insbesondere für die unmittelbare Herstellung der Gefäßwandung auf dem stabförmigen gewellten Kern eignen sich gemäß einer bevorzugten Ausführungsform solche Kerne, die im Durchmesser verkleinerbar sind oder auch zerlegbare Kerne. So ist es möglich, den Kern mehrteilig auszubilden, beispielsweise kann zur Ausbildung des Kerns ein zylindrischer Stab vorgesehen sein, der mit einer aufschiebbaren und abziehbaren Wendel entsprechender Größe kombiniert ist.

Weitere Merkmale der Erfindung ergeben sich aus der nachfolgenden Zeichnung in Verbindung mit den Unteransprüchen. Hierbei können die Merkmale jeweils für sich alleine oder in Kombination miteinander vorgesehen sein.

In den Zeichnungen zeigen
Figur 1: eine Ausführungsform einer erfindungsgemäßen plissierten non woven Gefäßprothese und
Figur 2: ein Herstellungsstadium, bei dem sich die Prothese noch auf einem stabförmigen Kern befindet.

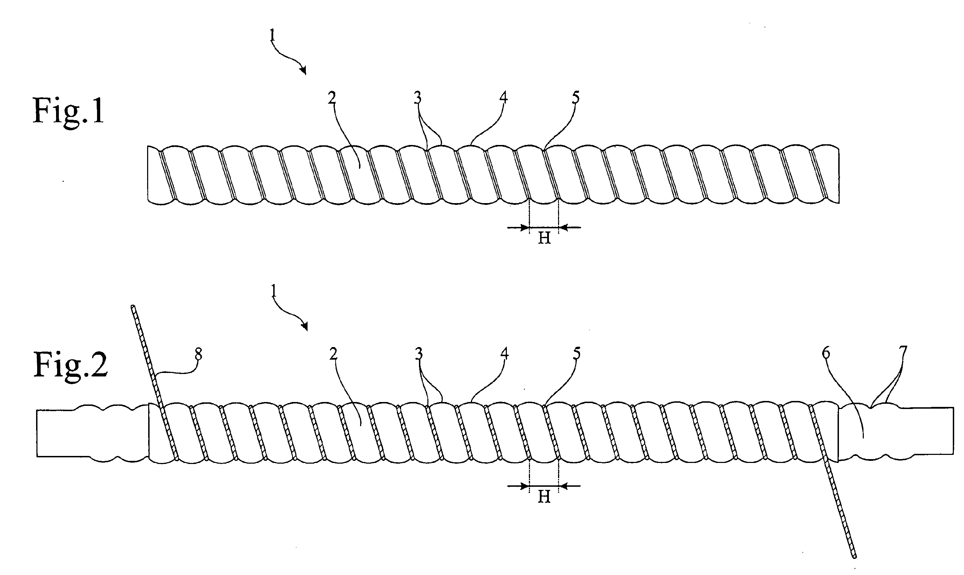

Bei der Ausführungsform nach Figur 1 ist eine non woven Gefäßprothese 1 aus einem Polyurethansprühvlies dargestellt, bei der die Gefäßwandung 2 als poröses Sprühvlies ausgebildet ist und eine wendelförmig verlaufende wellenförmige Plissierung 3 aufweist. Die Prothesenwandung 2 besteht aus einer Vielzahl von Polyurethanfasern, die eine dreidimensionale poröse Struktur bilden und miteinander verklebt sind. Die Porosität entspricht einer Luftdurchlässigkeit von 30 ml Luft pro Quadratzentimeter pro Minute bei einer Druckdifferenz von 1,2 KPas. Die Plissierung der Gefäßprothese besitzt eine Ganghöhe H von 2,7 mm. Die Wellung der Plissierung 3 ist asymmetrisch. Die konvexen, die Wellenberge bildenden Wölbungen 4 besitzen einen größeren Radius als die konkaven, die Wellentäler bildenden Rillen 5.

Die Gefäßprothesenwandung hat eine Wandstärke von 0,5 mm. Der lichte Innendurchmesser der Gefäßprothese beträgt 5 mm. Der Außendurchmesser beträgt im Bereich der Wellenberge 6,0 mm und im Bereich der Vertiefungen 5,5 mm.

Die Gefäßprothese lässt sich um enge Radien biegen, ohne zu kollabieren. Beim radialen Zusammendrücken ist die Rückstellkraft der plissierten Gefäßprothese deutlich größer als bei einer unplissierten Gefäßprothese.

Hergestellt werden kann die erfindungsgemäße Prothese, indem wie in Figur 2 gezeigt eine vorgefertigte poröse non woven Gefäßprothese in Form eines zylindrischen Schlauches auf einen Stab 6 mit einer der gewünschten Plissierung entsprechenden wendelförmigen Wellung 7 aufgeschoben wird. Die Prothese wird kurz erwärmt und schmiegt sich durch Schrumpfung im Durchmesser der Wellenform des Stabes etwas an, wobei die Stabwendel an der Schlauchoberseite sichtbar wird. Ein Faden 8, z.B. aus Polyester, wird dann um die Prothesenwandung entsprechend der Wendelform des Stabes gewickelt, so dass die Schlauchwandung wendelförmig in die Vertiefungen der Stabwendel eingedrückt wird. Durch eine thermische Fixierung bei 50 °C nimmt der Schlauch die Wellenform des Stabes an, die nach dem Abkühlen bleibt. Der Stab kann nach dem Entfernen des Fadens 8 aus der plissierten Prothese mühelos herausgezogen werden.

Bei einer anderen Ausführungsform zur Herstellung der erfindungsgemäßen Prothese wird ein im Durchmesser veränderbarer oder zerlegbarer Kern verwendet. Bei dieser Ausführungsform können Kerne vorgesehen sein, bei denen keilförmige oder konische Innenstücke aus dem Kern unter Durchmesserverringerung herausziehbar sind oder die Wendel auf einem stabförmigen zylindrischen Kern verschiebbar ausgebildet ist. Insbesondere bei Verwendung solcher Kerne kann die Prothese in plissierter Form auch unmittelbar auf dem Kern, beispielsweise durch Tauchen oder aufbauendes Sprühen, hergestellt werden.

## Patentansprüche

1. Non woven Gefäßprothese (1) mit einer Plissierung (3) in der Gefäßwandung (2), wobei die Gefäßwandung (2) von einem Vlies gebildet wird, **dadurch gekennzeichnet, dass** die Gefäßwandung (2) aus Polyurethan gebildet ist und das Polyurethan ein thermoplastisches und in Lösungsmittel lösliches Polyurethan ist.

2. Non woven Gefäßprothese (1) nach Anspruch 1, **dadurch gekennzeichnet, dass** die Plissierung (3) wellenförmig ausgebildet ist.

3. Non woven Gefäßprothese nach einem der Ansprüche 1 oder 2, **dadurch gekennzeichnet, dass** die Gefäßwandung (2) porös ist und gegebenenfalls mit einer resorbierbaren Imprägnierung abgedichtet ist.

4. Non woven Gefäßprothese nach einem der vorhergehenden Ansprüche, **dadurch gekennzeichnet, dass** die Gefäßwandung (2) von einem Sprühvlies gebildet wird.

5. Non woven Gefäßprothese nach einem der vorhergehenden Ansprüche, **dadurch gekennzeichnet, dass** die Plissierung (3) Rillen (5) aufweist, die vorzugsweise wendelförmig entlang der Gefäßwandung (2) verlaufen.

6. Non woven Gefäßprothese nach einem der vorhergehenden Ansprüche, **dadurch gekennzeichnet, dass** Vertiefungen, insbesondere Rillen (5), der Plissierung (3) von Einschnürungen gebildet werden.

7. Non woven Gefäßprothese nach Anspruch 6, **dadurch gekennzeichnet, dass** die Gefäßwandung (2) im Bereich der Vertiefungen (5), insbesondere durch Einschnürungen, verdichtet ist.

8. Non woven Gefäßprothesen nach einem der vorhergehenden Ansprüche, **dadurch gekennzeichnet, dass** der Innendurchmesser der Prothese 2 bis 40 mm, insbesondere 4 bis 12 mm, beträgt.

9. Non woven Gefäßprothese nach einem der vorhergehenden Ansprüche, **dadurch gekennzeichnet, dass** die Wandstärke der Gefäßwandung (2) 0,2 bis 1 mm, insbesondere 0,4 bis 0,6 mm, beträgt.

10. Non woven Gefäßprothese nach einem der vorhergehenden Ansprüche, **dadurch gekennzeichnet, dass** die Plissierung (3) eine Rillentiefe von 0,2 bis 1 mm, insbesondere 0,4 bis 0,6 mm, besitzt.

11. Verfahren zur Herstellung der plissierten non woven Gefäßprothese nach einem der vorhergehenden Ansprüche, **dadurch gekennzeichnet, dass** die Gefäßwandung an einem stabförmigen Kern mit einer der Plissierung entsprechenden gewellten Oberfläche abgeformt wird.

12. Verfahren nach Anspruch 11, **dadurch gekennzeichnet, dass** ein stabförmiger Kern mit einer wendelförmig umlaufenden Wellung auf seiner Oberfläche eingesetzt wird.

13. Verfahren nach Anspruch 11 oder 12, **dadurch gekennzeichnet, dass** eine vorgefertigte, noch nicht plissierte non woven Gefäßprothese zur Ausbildung der Plissierung auf den stabförmigen Kern aufgeschoben wird und die Plissierung durch querschnittsverjüngende, insbesondere thermische Fixierung ausgebildet wird.

14. Verfahren nach einem der Ansprüche 11 bis 13, **dadurch gekennzeichnet, dass** die vorgefertigte Gefäßprothese auf dem stabförmigen Kern entsprechend der wellenförmigen Oberfläche in den Wellentälern eingeschnürt wird und die Einschnürungen fixiert werden.

15. Verfahren nach einem der Ansprüche 11 bis 14, **dadurch gekennzeichnet, dass** eine im Querschnitt reversibel vorgedehnte vorgefertigte Gefäßprothese auf den stabförmigen Kern aufgeschoben und auf diesen aufgeschrumpft wird.

16. Verfahren nach einem der Ansprüche 11 bis 15, **dadurch gekennzeichnet, dass** auf den stabförmigen Kern, insbesondere zwischen den stabförmigen Kern und die vorgefertigte Gefäßprothese, eine gleitfähige Zwischenschicht, insbesondere eine Folie aufgebracht wird.

17. Verfahren nach einem der Ansprüche 11, 12 oder 16, **dadurch gekennzeichnet, dass** die Prothese mit Plissierung durch Aufsprühen einer Lösung von Polyurethan auf den stabförmigen Kern als Sprühvlies mit gewellter Oberfläche ausgebildet wird.

18. Verfahren nach einem der Ansprüche 11 bis 17 **dadurch gekennzeichnet, dass** ein im Querschnitt verringerbarer Kern mit gewellter Oberfläche eingesetzt wird und der Querschnitt des Kerns zum erleichterten Abziehen der plissierten Gefäßprothese verringert wird.

19. Verfahren nach einem der Ansprüche 11 bis 18, **dadurch gekennzeichnet, dass** die gewellte Oberfläche des Kerns von einer auf einen Stab gewickelten, vorzugsweise in Längsrichtung abziehbaren, Wendel gebildet wird.

## Claims

1. A nonwoven vascular prosthesis (1) with pleats (3) in the vessel wall (2), wherein the vessel wall (2) is made of a nonwoven fabric, **characterized in that** the vessel wall (2) is made of polyurethane and **in that** the polyurethane is a thermoplastic polyurethane and soluble in solvent.

2. The nonwoven vascular prosthesis (1) according to claim 1, **characterized in that** the pleats (3) are in the form of waves.

3. The nonwoven vascular prosthesis according to any of claims 1 or 2, **characterized in that** the vessel wall (2) is porous and may be sealed using a resorbable impregnating agent if required.

4. The nonwoven vascular prosthesis according to any one of the preceding claims, **characterized in that** the vessel wall (2) is formed from a sprayed non-woven web.

5. The nonwoven vascular prosthesis according to any one of the preceding claims, **characterized in that** the pleats (3) have grooves (5), which preferably run helically along the vessel wall (2).

6. The nonwoven vascular prosthesis according to any one of the preceding claims, **characterized in that** furrows, in particular grooves (5), of the pleats (3) are formed by constricted zones.

7. The nonwoven vascular prosthesis according to claim 6, **characterized in that** the vessel wall (2) in the region of the furrows (5) is compacted, in particular by constricted zones.

8. The nonwoven vascular prosthesis according to any one of the preceding claims, **characterized in that** the internal diameter of the prosthesis is 2 to 40 mm, in particular 4 to 12 mm.

9. The nonwoven vascular prosthesis according to any one of the preceding claims, **characterized in that** the wall thickness of the vessel wall (2) is 0.2 to 1 mm, in particular 0.4 to 0.6 mm.

10. The nonwoven vascular prosthesis according to any one of the preceding claims, **characterized in that** the pleats (3) have a groove depth of 0.2 to 1 mm, in particular 0.4 to 0.6 mm.

11. A method for producing the pleated, nonwoven vascular prosthesis according to any one of the preceding claims, **characterized in that** the vessel wall is formed on a rod-shaped core having a corrugated surface corresponding to the pleats.

12. The method according to claim 11, **characterized in that** a rod-shaped core having a helically encircling, corrugated construction on its surface is used.

13. The method according to claim 11 or 12, **characterized in that** a prefabricated, yet unpleated, nonwoven vascular prosthesis is pushed onto the rod-shaped core to produce the pleats and the pleats are formed by treatment which causes a reduction in size of the cross-section, in particular by heat-setting.

14. The method according to any one of the claims 11 to 13, **characterized in that** the prefabricated vascular prosthesis on the rod-shaped core is constricted in the wave troughs of the corrugated surface, and the constricted zones are fixed in place.

15. The method according to any one of the claims 11 to 14, **characterized in that** a prefabricated vascular prosthesis, reversibly pre-stretched in the cross-section, is pushed onto the rod-shaped core and shrunk onto it.

16. The method according to any one of the claims 11 to 15, **characterized in that** a slippery intermediate layer, in particular a film, is applied on the rod-shaped core, in particular between the rod-shaped core and the prefabricated vascular prosthesis.

17. The method according to any one of the claims 11, 12 or 16, **characterized in that** the prosthesis is produced with pleats by spraying a solution of polyurethane onto the rod-shaped core to form a sprayed non-woven web with a corrugated surface.

18. The method according to any one of the claims 11 to 17, **characterized in that** a core, having a corrugated surface and a cross-section that is reducible in size, is used and the cross-section of the core is reduced to facilitate removal of the pleated vascular prosthesis.

19. The method according to any one of the claims 11 to 18, **characterized in that** the corrugated surface of the core is formed by a helix, which is wound onto a rod, and preferably is removable in the lengthwise direction.

## Revendications

1. Prothèse vasculaire non tissée (1) avec un plissage (3) dans la paroi vasculaire (2), dans laquelle la paroi vasculaire (2) est formée par un non-tissé, **caractérisée en ce que** la paroi vasculaire (2) est formée de polyuréthane et le polyuréthane est un polyuréthane thermoplastique et soluble dans un solvant.

2. Prothèse vasculaire non tissée (1) selon la revendication 1, **caractérisée en ce que** le plissage (3) est réalisé sous forme ondulée.

3. Prothèse vasculaire non tissée selon l'une des revendications 1 ou 2, **caractérisée en ce que** la paroi vasculaire (2) est poreuse et est éventuellement rendue étanche avec une imprégnation résorbable.

4. Prothèse vasculaire non tissée selon l'une quelconque des revendications précédentes, **caractérisée en ce que** la paroi vasculaire (2) est formée par un non-tissé pulvérisé.

5. Prothèse vasculaire non tissée selon l'une quelconque des revendications précédentes, **caractérisée en ce que** le plissage (3) présente des stries (5), qui s'étendent de préférence en forme d'hélice le long de la paroi vasculaire (2).

6. Prothèse vasculaire non tissée selon l'une quelconque des revendications précédentes, **caractérisée en ce que** des creux, en particulier des stries (5), du plissage (3) sont formés par des rétrécissements.

7. Prothèse vasculaire non tissée selon la revendication 6, **caractérisée en ce que** la paroi vasculaire (2) est comprimée dans la région des creux (5), en particulier par des rétrécissements.

8. Prothèse vasculaire non tissée selon l'une quelconque des revendications précédentes, **caractérisée en ce que** le diamètre intérieur de la prothèse vaut 2 à 40 mm, en particulier 4 à 12 mm.

9. Prothèse vasculaire non tissée selon l'une quelconque des revendications précédentes, **caractérisée en ce que** l'épaisseur de paroi de la paroi vasculaire (2) vaut 0,2 à 1 mm, en particulier 0,4 à 0,6 mm.

10. Prothèse vasculaire non tissée selon l'une quelconque des revendications précédentes, **caractérisée en ce que** le plissage (3) présente une profondeur de stries de 0,2 à 1 mm, en particulier de 0,4 à 0,6 mm.

11. Procédé de fabrication de la prothèse vasculaire non tissée plissée selon l'une quelconque des revendications précédentes, **caractérisé en ce que** l'on façonne la paroi vasculaire sur un noyau en forme de barre avec une surface ondulée correspondant au plissage.

12. Procédé selon la revendication 11, **caractérisé en ce que** l'on utilise un noyau en forme de barre avec une ondulation périphérique en forme d'hélice à sa surface.

13. Procédé selon la revendication 11 ou 12, **caractérisé en ce que** l'on enfile une prothèse vasculaire non tissée préfabriquée pas encore plissée sur le noyau en forme de barre pour former le plissage et on forme le plissage par une fixation, en particulier thermique, réduisant la section transversale.

14. Procédé selon l'une quelconque des revendications 11 à 13, **caractérisé en ce que** l'on rétrécit la prothèse vasculaire préfabriquée dans les vallées de l'ondulation sur le noyau en forme de barre selon la surface ondulée correspondante et l'on fixe les rétrécissements.

15. Procédé selon l'une quelconque des revendications 11 à 14, caractérisé en ce l'on enfile une prothèse vasculaire préfabriquée pré-étendue de façon réversible en section transversale sur le noyau en forme de barre et on la rétracte sur celui-ci.

16. Procédé selon l'une quelconque des revendications 11 à 15, **caractérisé en ce que** l'on dépose une couche intermédiaire glissante, en particulier une pellicule, sur le noyau en forme de barre, en particulier entre le noyau en forme de barre et la prothèse vasculaire préfabriquée.

17. Procédé selon l'une des revendications 11, 12 ou 16, **caractérisé en ce que** l'on forme la prothèse avec un plissage par pulvérisation d'une solution de polyuréthane sur le noyau en forme de barre sous forme de non-tissé pulvérisé avec une surface ondulée.

18. Procédé selon l'une quelconque des revendications 11 à 17, **caractérisé en ce que** l'on utilise une noyau avec une surface ondulée et dont la section transversale peut être réduite et on réduit la section transversale du noyau pour faciliter l'enlèvement de la prothèse vasculaire plissée.

19. Procédé selon l'une quelconque des revendications 11 à 18, **caractérisé en ce que** l'on forme la surface ondulée du noyau au moyen d'une hélice enroulée sur une barre, pouvant être retirée de préférence en direction longitudinale.
